# EUROPÄISCHE PATENTSCHRIFT

(11) **EP 0 721 768 B1**
(45) Veröffentlichungstag und Bekanntmachung des Hinweises auf die Patenterteilung: **12.12.2001**
(21) Anmeldenummer: 96100429.8
(22) Anmeldetag: 12.01.1996
(51) Int. Cl.: A61F 13/00, A61F 13/15

(54) **Wegwerfwindel mit einem abgeschrägten Randabschnitt**
Disposable diaper with a sloped edge section
Couche jetable à bord en biseau

(30) Priorität: 13.01.1995 JP 437395
(43) Veröffentlichungstag der Anmeldung: 17.07.1996
(73) Patentinhaber: UNI-CHARM CORPORATION, Kawanoe-shi Ehime-ken (JP)
(72) Erfinder: Inoue, Yasushi, Kannonji-shi, Kagawa-ken (JP); Kido, Tsutomu, Kawanoe-shi, Ehime-ken (JP)
(74) Vertreter: Sperling, Rüdiger, Dipl.-Ing.

(56) Entgegenhaltungen:
- EP-A- 0 235 854
- EP-A- 0 572 033
- EP-A- 0 596 532
- GB-A- 2 103 930
- US-A- 4 519 798
- US-A- 4 938 754

## Beschreibung

Die vorliegende Erfindung betrifft eine Wegwerfwindel, enthaltend eine flüssigkeitsdurchlässige Decklage, eine flüssigkeitsundurchlässige Außenlage und eine flüssigkeitsabsorbierende Füllung mit einem flüssigkeitsabsorbierenden Kern und erste und zweite flüssigkeitsabsorbierende Lagen, die die Oberseite bzw. die Unterseite des Kerns bedecken, wobei die Randabschnitte der ersten und zweiten flüssigkeitsabsorbierenden Lagen, die von einer Umfangskante des Kerns horizontal auswärts verlaufen, in einem vorgegebenen Abstand (d) von der Umfangskante des Kerns flach zusammengelegt und miteinander verbunden sind und eine innere Hülle bilden und wobei die Randabschnitte der Deck- und Außenlagen, die sich auswärts über die Umfangskante der Füllung hinaus erstrecken und in einem vorgegebenen Abstand (D) von der Umfangskante des Kerns flach zusammengelegt und somit miteinander verbunden sind, dass sie eine äußere Hülle bilden, wobei die Randabschnitte der ersten und zweiten Flüssigkeit absorbierenden Lagen zwischen den Randabschnitten der Deck- und Außenlagen angeordnet sind.

Herkömmliche Wegwerfwindeln enthalten eine flüssigkeitsabsorbierende Füllung mit einem Kern, der aus einer Mischung aus einer bauschigen Pulpe und superabsorbierenden Polymerteilchen formgepreßt ist, und einer flüssigkeitsdurchlässigen Lage, wie ein Seiden- und gewebeartiges Papier, die zum Abdecken des Kerns ausgelegt ist. Zum Beispiel sind in einer in dem US-Patent Nr. 4,636,207 offenbarten Windel Ober- und Unterseiten eines absorbierenden Kerns mit Ober- bzw. Unterseiten-Seidenpapieren bedeckt. Gemäß dieser bekannten Windel verläuft das Unterseiten-Seidenpapier über in Längsrichtung entgegengesetzte Enden des Kerns hinaus, und dann sind diese Teile, die über die Enden des Kerns hinaus verlaufen, längs den Enden zurück auf die Oberseite des Kerns gefaltet, um mit dem Oberseiten-Seidenpapier verbunden zu sein. Bei sowohl den Ober- als auch den Unterseiten-Seidenpapieren sind die Breiten schmäler als jene des Kerns, und daher liegen die in Querrichtung entgegengesetzten Seitenkanten dieser Seiden- oder gewebeartigen Papiere innerhalb der seitlichen Kernseitenkanten. Eine flüssigkeitsabsorbierende Füllung, deren Kern in dieser Weise mit den Ober- und Unterseiten-Seidenpapieren bedeckt ist, ist dann von einer flüssigkeitsdurchlässigen Deckschicht und einer flüssigkeitsundurchlässigen Außenschicht umgeben. Die Ober- und Unterseiten-Seidenpapiere sind jeweils mit dem Kern und ebenfalls mit den Deck- und Außenschichten verbunden, um zu verhindern, dass sich die flüssigkeitsabsorbierende Füllung relativ zu diesen Deck- und Außenschichten verlagert.

Nach dem US-Patent Nr. 4,681,579 enthält eine Windel eine flüssigkeitsabsorbierende Füllung, die einen Kern hat, der mit einem einzigen Tissue- oder Seidenpapier umgeben ist, das den Kern längs seiner Oberseite, einer seiner seitlich entgegengesetzten Seitenkanten, seiner Unterseite und der anderen seiner seitlich entgegengesetzten Seitenkanten ohne Unterbrechung umwickelt. Ferner ist bei dieser Windel das Seiden- oder gewebeartige Papier in engem Kontakt mit den seitlich entgegengesetzten Seitenkanten des Kerns.

Die US 4,519,798 zeigt eine Wegwerfwindel oder eine Binde, die ebenfalls aus einem Kern, einer inneren und einer äußeren Hülle gebildet wird. Dabei umschließt allerdings die äußere Hülle die innere Hülle nicht vollständig, so dass die außen liegenden Endbereiche der inneren und äußeren Hülle zwar gemeinsam verschweißt werden können, wobei allerdings kein weicher flexibler Übergang zwischen dem zentralen Bereich der Windel und den Endbereichen durch getrennte Auslegung der Verschweißungsbereiche der inneren und äußeren Hülle gewährleistet wird.

Die GB 2 103 930 zeigt ebenfalls eine Wegwerfwindel mit ähnlichem Aufbau, in der allerdings keinerlei Angaben über die Ausgestaltung der Randbereiche im Hinblick auf einen möglichst flexiblem Übergang zu entnehmen sind.

Die EP 0 596 532 offenbart ein mehrschichtiges Material zur Verwendung in Windeln und ein Herstellungsverfahren desselben, das einen vielschichtigen Aufbau zeigt, der sich allerdings in Längs- und Querrichtung unterscheidet.

Die US 4,938,754 und die EP 0 572 033 zeigen eine Wegwerfwindel bzw. eine Binde mit einem mehrschichtigen, komplexen Aufbau, dessen Randbereiche sich jeweils in Längs- und Querrichtung unterscheiden.

Die Anordnung des Kerns, der mit dem bzw. den Tissue- oder Seidenpapier(en) bedeckt ist, wie es in den oben angegebenen Literaturstellen offenbart ist, ist sicherlich wirksam, eine Deformation des Kerns an seinen seitlich entgegengesetzten Seitenkanten, in Längsrichtung entgegengesetzten Enden und den Stellen zu verhindern, die mit dem bzw. den Seidenpapier(en) eng oder dicht bedeckt sind. Jedoch sollte bedacht werden, dass der Kern oft aus einer Mischung aus einer bauschigen Pulpe und superabsorbierenden Polymerteilchen formgepreßt ist und eine relativ große Steifigkeit hat. Wenn die bekannten Abdeck- oder Umhüllungsmittel, wie sie oben angegeben sind, bei einem derartigen Kern angewandt werden, wird eine Umfangskante des Kerns ziemlich ekkig oder scharf sein und oft einen Tragekomfort wegen seines steifen Anfühlens verschlechtern. Die Verbindungsmittel der bzw. des Seidenpapiere(s) zum Kern sowie der Deck- und Außenlage werden ebenfalls das steife Anfühlen verursachen und oft einen Tragekomfort verschlechtern.

Im Hinblick auf die Probleme, wie sie oben beschrieben wurden, ist es ein grundsätzliches Ziel der vorliegenden Erfindung, derartige Probleme zu lösen.

Dieses Ziel wird durch eine Wegwerfwindel gelöst, bei der die äußere Hülle die innere Hülle vollständig umschließt und bei der das Verhältnis der Abstände von D zu d: "D ≥ d" beträgt.

Vorteilhafte und bevorzugte Weiterbildungen der Erfindung ergeben sich aus den Unteransprüchen im einzelnen und deren Kombination.

Hauptsächlich werden die oben angegebenen Probleme dadurch gelöst, dass erste und zweite flüssigkeitsabsorbierende Lagen, die Ober- bzw. Unterseiten eines Kerns bedecken, längs ihrer Umfangsränder auswärts beabstandet von einer Umfangskante des Kerns in einem vorgegebenen oder gewünschten Abstand flach miteinander verbunden sind.

Das oben angegebene Ziel wird gemäß der Erfindung durch eine verbesserte Wegwerfwindel erreicht, die eine flüssigkeitsdurchlässige Decklage, eine flüssigkeitsundurchlässige Außenlage und eine flüssigkeitsabsorbierende Füllung enthält, die einen flüssigkeitsabsorbierenden Kern und erste und zweite flüssigkeitsabsorbierende Lagen hat, die die Ober- bzw. Unterseiten des Kerns bedecken, wobei die Verbesserung umfaßt, dass Randabschnitte der ersten und zweiten flüssigkeitsabsorbierenden Lagen, die von einer Umfangskante des Kerns horizontal auswärts verlaufen, in einem vorgegebenen oder gewünschten Abstand von der Umfangskante des Kerns flach zusammengelegt und miteinander verbunden sind.

Gemäß einer Ausführung der Erfindung sind Randabschnitte der Deck- und Außenlagen, die sich auswärts über die Umfangskante des Kerns hinaus erstrecken, so miteinander verbunden, dass sie eine Hülle bilden, und eine Außenkante der Randabschnitte der ersten und zweiten flüssigkeitsabsorbierenden Lagen, die flach zusammengelegt und miteinander verbunden sind, ist horizontal gegen eine Innenseite der Hülle angelegt.

Gemäß einer weiteren Ausführung der Erfindung sind die ersten und zweiten flüssigkeitsabsorbierenden Lagen zwischen den Randabschnitten der Deck- und Außenlagen angeordnet, die sich horizontal auswärts über die Umfangskante des Kerns hinaus erstrecken.

Bei der Windel, die gemäß der obigen Beschreibung aufgebaut ist, bestimmt wenigstens eine der ersten und zweiten flüssigkeitsabsorbierenden Lagen eine sanfte Schräge oder Neigung zwischen der Umfangskante des Kerns und den Umfangsrändern der ersten und zweiten flüssigkeitsabsorbierenden Gewebe- und Tissue-Lagen, die in einem erwünschten Abstand von der Umfangskante des Kerns flach miteinander verbunden sind, und diese Schräge wirkt so, dass verhindert wird, dass die Umfangskante eckig oder steif wird.

Um zu verhindern, dass sich die Füllung relativ zu den Deck- und Außenlagen verlagert, sind die Umfangsränder der Deck- und Außenlagen miteinander verbunden, um eine Hülle zu bilden, und die Außenränder, längs denen die ersten und zweiten flüssigkeitsabsorbierenden Lagen verbunden sind, liegen horizontal gegen die Innenseite der Hülle an, oder die ersten und zweiten flüssigkeitsabsorbierenden Lagen sind zwischen den Umfangsrändern der Deck- und Außenlagen angeordnet und diese Umfangsränder sind miteinander verbunden.

Die Erfindung wird nachfolgend anhand von Ausführungsbeispielen unter Bezugnahme auf die zugehörigen Zeichnungen näher beschrieben, in denen:
Fig. 1 eine perspektivische Ansicht ist, die eine Ausführung einer Wegwerfwindel gemäß der vorliegenden Erfindung in teilweise aufgebrochener Ansicht zeigt;
Fig. 2 eine Schnittansicht längs einer Linie II-II in der Fig. 1 ist;
Fig. 3 eine Schnittansicht längs einer Linie III-III in der Fig. 1 ist; und
Fig. 4 eine eine Variante der Erfindung zeigende Ansicht ähnlich der Fig. 2 ist.

Eine Wegwerfwindel gemäß der Erfindung ist anhand der folgenden Beschreibung unter Bezugnahme auf die Zeichnungen besser zu verstehen.

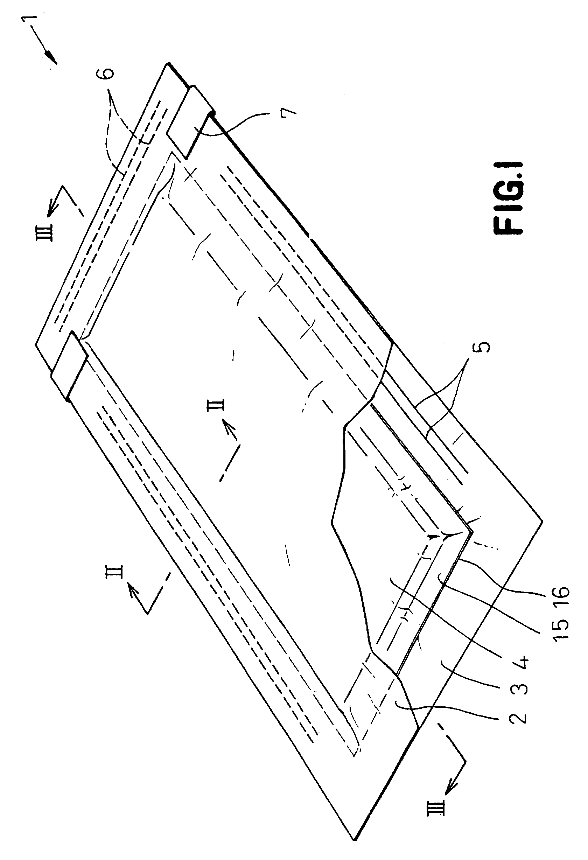

Unter Bezugnahme auf die Fig. 1, 2 und 3 enthält eine Windel 1 eine flüssigkeitsdurchlässige Decklage 2, eine flüssigkeitsundurchlässige Außenlage 3 und eine flüssigkeitsabsorbierende Füllung 4, die zwischen diesen beiden Lagen 2, 3 angeordnet ist. Die Windel 1 ist längs ihren querverlaufenden, entgegengesetzten Seitenrändern und längs eines oberen Endes eines Hinterteils mit einem Paar von Elastikelementen 5 für beide Beinöffnungen bzw. einem Elastikelement 6 für die Hüftöffnung versehen. Diese Elastikelemente 5, 6 sind unter Spannung mit einer Innenfläche der Deck- oder Außenlage 2, 3 verbunden. Das Hinterteil ist an quer entgegengesetzten Seitenrändern mit einem Paar von Befestigungsbändern 7 mit wohlbekannter Technik versehen.

Die Deck- und Außenlagen 2, 3 verlaufen horizontal über eine Umfangskante eines rechtwinkligen Kerns 14 der Füllung 4 hinaus und sind in einem Abstand "D" von der Umfangskante des Kerns 14 flach zusammengelegt und durch Heißkleber oder thermoplatischen Klebstoff 11 miteinander verbunden, so daß sie eine umfangsmäßig abgedichtete Außenhülle 12 bilden.

Die Füllung 4 enthält den Kern 14, der aus einer Mischung aus einer flockigen, bauschige Pulpe und super- oder hochabsorbierenden Polymerteilchen formgepreßt ist, und eine flüssigkeitsabsorbierende Oberseitenlage 15, die aus TissuePapier oder Textilverbundstoffen hergestellt und zum Bedekken einer Oberseite des Kerns 14 ausgelegt ist, und eine flüssigkeitsabsorbierende Unterseitenlage 16, die aus Tissue-Papier oder Textilverbundstoffen hergestellt und zum Bedecken einer Unterseite des Kerns 14 ausgelegt ist. Diese Ober- und Unterseitenlagen 15, 16 haben die gleiche rechtwinklige Größe, die sich auswärts über die Umfangskante des Kerns 14 hinaus erstreckt, und sind in einem Abstand "d" von der Umfangskante des Kerns 14 durch Heißkleber oder thermoplatischen Klebstoff 17 flach miteinander verbunden, um eine Innenhülle 18 zu bilden. Eine Außenkante dieser Innenhülle 18 ist in horizontaler Gegenüberlage zu einer Innenseite der Außenhülle 12 angeordnet, so daß sich die Füllung 4 nicht ohne weiteres innerhalb der Außenhülle 12 verlagern oder verschieben kann. Es dürfte verständlich sein, daß der Kern 14 notfalls intermittierend mit den Ober- und Unterseitenlagen verbunden sein kann, um sie an einer Relativverlagerung zu hindern.

Die Deck- und Außenlagen 2, 3, die die Außenhülle 12 bilden, und die Ober- und Unterseitenlagen 15, 16 bestimmen über die Abstände "D" bzw. "d" von der Umfangskante des Kerns 14 sanfte Schrägen oder Neigungen, die die relativ steifen Seitenkanten des Kerns 14 daran hindern, in die Haut eines Trägers einzuschneiden. Eine Relation zwischen dem Abstand "D" und dem Abstand "d" kann sein: "D" > "d", solange sie an der selben Stelle gemessen werden. Werte dieser Abstände können von den jeweiligen Seitenrändern und Enden in Längsrichtung abhängen.

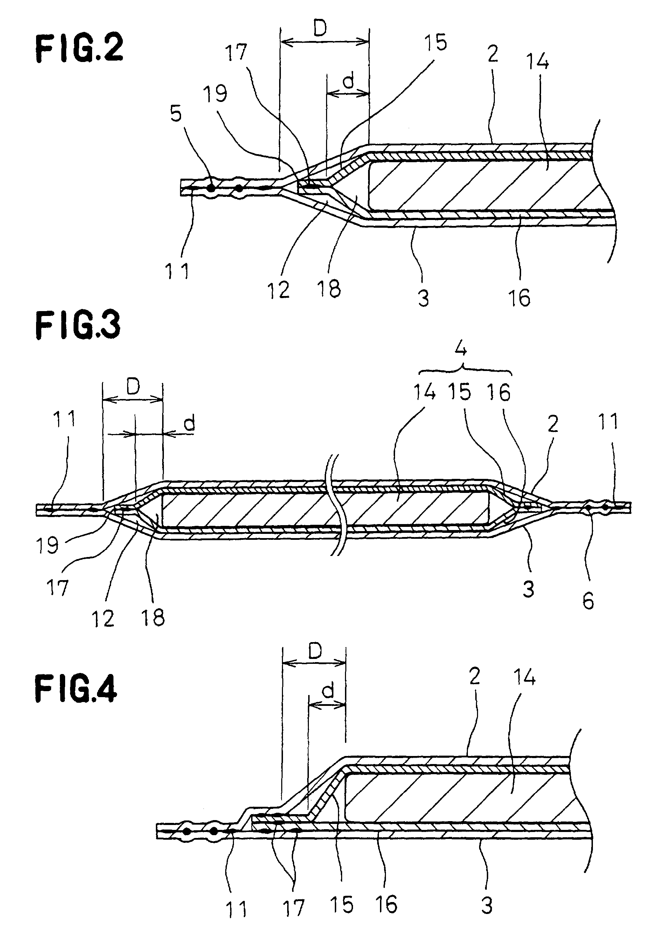

Unter Bezugnahme auf die Fig. 4 sind in der Windel 1 die Umfangsränder der Ober- und Unterseitenlagen 15, 16 angeordnet und verbunden zwischen den Umfangsrändern der Deck- und Außenlagen 2, 3, die flach aneinander gelegt sind, um sicherzustellen, daß sich die Füllung 4 nicht relativ zu den Deck- und Außenlagen 2, 3 verlagern oder verschieben kann. In diesem Fall sind die Ober- und Unterseitenlagen 15, 16 ebenfalls vorzugsweise mittels eines Heißklebers 17 miteinander verbunden. Diese durch die Fig. 4 gezeigte Variante unterscheidet sich von der Ausführung der Fig. 2. Es kann eine Relation "D" = "d" eingestellt sein.

Für die Windel 1 der Erfindung können die Deck- und Außenlagen 2, 3 sowie die Ober- und Unterseitenlagen 15, 16 aus Materialien bestehen, die üblicherweise in diesem technischen Gebiet eingesetzt werden. Die Ober- und Unterseitenlagen 15, 16 können hydrophile Fasern gemischt mit einer erwünschten Menge von hydrophoben Fasern enthalten, um ein Körperflüssigkeits-Diffusionsvermögen dieser Lagen zu verbessern.

Bei der Windel der Erfindung sind die Umfangskante des Kerns weder eckig noch scharf und der Tragekomfort der Windel verbessert, da sich die Ober- und Unterseitenlagen auswärts über die Umfangskante des Kerns hinaus erstrecken und in einem gewünschten oder vorgesehenen Abstand von der Umfangskante des Kerns flach miteinander verbunden sind.

Die erfindungsgemäße Windel macht es in vorteilhafter Weise unnötig, die Ober- und Unterlagen mittels eines Klebers an die Deck- und Außenlagen anzubinden, wie es beim Stand der Technik erforderlich war, und verwirklicht dadurch eine weiche Berührung der Windel, da die Außenränder der Ober- und Unterseitenlagen horizontal gegen die Innenseiten der Außenhülle plaziert sind, die durch die Deck- und Außenlagen gebildet ist, um die Füllung daran zu hindern, sich relativ zu den Deck- und Außenlagen zu verschieben oder zu verlagern.

Zusammenfassend wird durch die Erfindung eine Wegwerfwindel geschaffen, bei der zwischen einer flüssigkeitsdurchlässigen Decklage und einer flüssigkeitsundurchlässigen Außenlage eine flüssigkeitsabsorbierende Füllung angeordnet ist, bei der ein flüssigkeitsabsorbierender Kern mit ersten und zweiten flüssigkeitsabsorbierenden Lagen bedeckt ist, die in einem vorgegebenen Abstand von einer Umfangskante des Kerns flach zusammengelegt und miteinander verbunden sind.

## Patentansprüche

1. Wegwerfwindel (1), enthaltend eine flüssigkeitsdurchlässige Decklage (2), eine flüssigkeitsundurchlässige Außenlage (3) und eine flüssigkeitsabsorbierende Füllung (4) mit einem flüssigkeitsabsorbierenden Kern (14) und ersten und zweiten flüssigkeitsabsorbierenden Lagen (15, 16), die die Oberseite bzw. die Unterseite des Kerns (14) bedecken, wobei die Randabschnitte der ersten und zweiten flüssigkeitsabsorbierenden Lagen (15, 16), die von einer Umfangskante des Kerns (14) horizontal auswärts verlaufen, in einem vorgegebenen Abstand (d) von der Umfangskante des Kerns (14) flach zusammengelegt und miteinander verbunden sind und eine innere Hülle (18) bilden und wobei die Randabschnitte der Deck- und Außenlagen (2,3), die sich auswärts über die Umfangskante der Füllung (4) hinaus erstrekken und in einem vorgegebenen Abstand (D) von der Umfangskante des Kerns (14) flach zusammengelegt und so miteinander verbunden sind, daß sie eine äußere Hülle (12) bilden, wobei die Randabschnitte der ersten und zweiten flüssigkeitsabsorbierenden Lagen (15, 16) zwischen den Randabschnitten der Deck- und Außenlagen (2,3) angeordnet sind, **dadurch gekennzeichnet, daß** die äußere Hülle (12) die innere Hülle (18) vollständig umschließt, und daß das Verhältnis der Abstände von D zu d: "D ≥ d" beträgt.

2. Wegwerfwindel nach Anspruch 1, **dadurch gekennzeichnet, daß** eine Außenkante der Randabschnitte der ersten und zweiten flüssigkeitsabsorbierenden Lagen (15, 16) horizontal gegen eine Innenseite der Hülle (12) angelegt ist.

3. Wegwerfwindel nach Anspruch 1, **dadurch gekennzeichnet, daß** sich die Deck- und Außenlagen (2,3) horizontal auswärts über die Umfangskante des Kerns (14) hinaus erstrecken und flach miteinander verbunden sind.

4. Wegwerfwindel nach Anspruch 3, **dadurch gekennzeichnet, daß** die Randabschnitte der ersten und zweiten flüssigkeitsabsorbierenden Lagen (15,16) zwischen den Randabschnitten der Deck- und Außenlagen (2,3) verbunden sind.

5. Wegwerfwindel nach einem der vorhergehenden Ansprüche, **dadurch gekennzeichnet, daß** die ersten und zweiten flüssigkeitsabsorbierenden Lagen (15,16) aus gewebeartigem Papier oder faserigen Textilverbundstoffen hergestellt sind.

## Claims

1. Disposable diaper (1), including a liquid-permeable topsheet (2), a liquid-impermeable backsheet (3) and a liquid-absorbing filling (4) with a liquid-absorbing core (14) and first and second liquid-absorbing layers (15, 16) covering the top surface and the bottom surface of the core (14), respectively, wherein the edge sections of the first and second liquid-absorbing layers (15, 16) extending horizontally outwards from a peripheral edge of the core (14) are laid flat against each other at a predetermined distance (d) from the peripheral edge of the core (14) and are joined together forming an inner cover (18), and wherein the edge sections of the topsheet and the backsheet (2, 3) extending outwardly beyond the peripheral edge of the filling (4) are laid flat against each other at a predetermined distance (D) from the peripheral edge of the core (14) and are joined together in such a way that they form an outer cover (12), the edge sections of the first and second liquid-absorbing layers (15, 16) being placed between the edge sections of the topsheet and the backsheet (2, 3)
**characterized in that** the outer cover (12) completely encloses the inner cover (18) and that the relationship of the distances D to d is "D ≥ d".

2. Disposable diaper as claimed in claim 1, **characterized in that** an outer edge of the peripheral sections of the first and second liquid-absorbing layers (15, 16) is laid horizontally against an inner side of the cover (12).

3. Disposable diaper as claimed in claim 1, **characterized in that** the topsheet and the backsheet (2, 3) extend horizontally outward beyond the peripheral edge of the core (14) and are joined together flatly.

4. Disposable diaper as claimed in claim 3, **characterized in that** the edge sections of the first and second liquid-absorbing layers (15, 16) are joined together between the edge sections of the the topsheet and the backsheet (2, 3).

5. Disposable diaper as claimed in one of the preceding claims, **characterized in that** the first and second liquid-absorbing layers (15, 16) are made of fabric-like paper or fibrous nonwoven fabric.

## Revendications

1. Un lange jetable (1) se composant d'une couche de couverture perméable au liquide (2), une couche extérieure imperméable au liquide (3), une masse de remplissage absorbant (4) sous la forme d'un noyau absorbant le liquide (14) et une première et deuxième couche (15, 16), recouvrant respectivement le dessus et le dessous du noyau (14), de telle manière que les sections périphériques de la première et deuxième couche absorbante (15, 16), partant horizontalement vers l'extérieur depuis l'un des bords périphériques du noyau (14), selon une distance prédéterminée (d) du bord périphérique du noyau (14) sont assemblées à plat et reliées ensemble pour former une enveloppe intérieure (18); les sections périphériques de la couche de couverture (2) et de la couche extérieure (3) allant vers l'extérieur par le bord périphérique du remplissage (4) et selon une distance prédéterminée (D) du bord périphérique du noyau (14) sont assemblées à plat et reliées ensemble pour former une enveloppe extérieure (12), les bords périphériques de la première et deuxième couche absorbante (15, 16) étant disposées entre les bords périphériques de la couche de couverture (2) et de la couche extérieure (3), **caractérisé en ce que** l'enveloppe extérieure (12) enferme complètement l'enveloppe intérieure (18) et que le rapport des distances de D à d = « D ≥ d ».

2. Un lange jetable selon la revendication 1 ci-dessus, **caractérisé en ce que** l'un des bords extérieurs de la section périphérique de la première et deuxième couche absorbant le liquide (15, 16) est disposé horizontalement contre un côté intérieur de l'enveloppe extérieure (12).

3. Un lange jetable selon la revendication 1 ci-dessus, **caractérisé en ce que** la couche de couverture (2) et la couche extérieure (3) s'étendent horizontalement vers l'extérieur par l'un des bords périphériques du noyau (14) et sont reliées ensemble à plat.

4. Un lange jetable selon la revendication 3 ci-dessus, **caractérisé en ce que** les bords périphériques de la première et deuxième couche absorbant le liquide (15, 16) sont reliés entre les bords périphériques de la couche de couverture (2) et la couche extérieure (3).

5. Un lange jetable selon 1 'une des revendications ci-dessus, **caractérisé en ce que** la première et la deuxième couche absorbant le liquide (15, 16) sont fabriquées en papier à structure tissée ou dans un matériau de fibres textiles agglomérées.
